(19) Europäisches Patentamt
European Patent Office
Office européen des brevets

(11) Publication number: **0 271 586**
**A1**

(12) # EUROPEAN PATENT APPLICATION

published in accordance with Art. 158(3) EPC

(21) Application number: 87903741.4

(51) Int.Cl.³: **C 07 D 205/08**

(22) Date of filing: 05.06.87

Data of the international application taken as a basis:

(86) International application number:
PCT/JP87/00354

(87) International publication number:
WO87/07603 (17.12.87 87/28)

(30) Priority: 09.06.86 JP 131889/86

(43) Date of publication of application:
22.06.88 Bulletin 88/25

(84) Designated Contracting States:
AT BE CH DE FR GB IT LI LU NL SE

(71) Applicant: **SAGAMI CHEMICAL RESEARCH CENTER**
4-5, Marunouchi 1-chome
Chiyoda-ku Tokyo 100(JP)

(72) Inventor: **TERASHIMA, Shiro**
2-27-4, Kyodo
Setagaya-ku Tokyo 156(JP)

(72) Inventor: **ITO, Yoshio**
4-4-1, Nishi Ohnuma
Sagamihara-shi Kanagawa 229(JP)

(72) Inventor: **KAWABATA, Takeo**
3-16, Sakaemachi
Sagamihara-shi Kanagawa 228(JP)

(74) Representative: Baarslag, Aldert D. et al,
Nederlandsch Octrooibureau Johan de Wittlaan 15 P.O. Box 29720
NL-2502 LS Den Haag(NL)

(54) **-g(b)-LACTAM COMPOUNDS AND PROCESS FOR THEIR PREPARATION.**

(57) β-Lactam compounds represented by general formula (I), wherein $R^1$ and $R^2$ are unified to form an oxygen atom or, when one of them represents a hydrogen atom, the other represents a hydroxy group or a protected hydroxy group, $R^3$ represents a hydrogen atom or a protective group for a hydroxy group, and $R^4$ represents a hydrogen atom or a protective group for an amino group and a process for their preparation. The compounds are useful as starting materials for synthesizing carbapenem antibiotics.

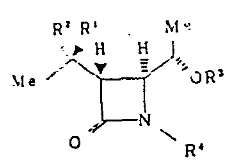

(I)

EP 0 271 586 A1

# DESCRIPTION

## β-lactam compounds and the method of their manufacture

Technical Field :

This invention concerns new β-lactam compounds and the method of their manufacture.

The compounds of this invention are useful as materials for the synthesizing of β-lactam derivatives which are expressed by general formula (II) below and which has acetoxy radicals at the 4-position:

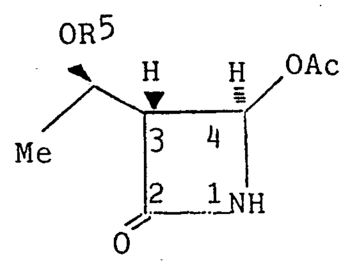

(II)

(where $R^5$ denotes hydrogen atom or hydroxyl radical-protecting groups).

The 4-acetoxy-β-lactam expressed by general formula (II) can be used as important intermediates for the synthesis of the tienamycin or other carbapenem antibiotics which have excellent antibacterial activity (T. Kametani, Heterocycles, 17, 463 (1982)).

Background Art:

Conventionally, as methods of synthesizing 4-acetoxy-β-lactam derivatives were known those where 6-aminopenicillanic acid, D-allo-threonine, L-threonine, L-aspartic acid and methyl 3-hydroxybutyrate were used as starting materials. (F. Di-Nnno,

et al, J.O.C., 42, 2960 (1977), M. Shiozaki et al, Terahedron, 39, 2399 (1983), H. Maruyama et al, Bull. Chem. Soc. Jpn., 58, 3264 (1985), P.J. Reider et al, Tetrahedron Lett., 23, 2293 (1982), T. Chiba et al, Chemistry Lett., 1985, 651)

These conventional methods, however, are expensive in materials, have too many manufacturing processes, and involve the oxidizing process where lead tetraacetate being problematical in the treatment of wastes after reactions is used. These methods, therefore, are attended with a great many difficulties in their commercial application. The inventors made a series of assiduous studies to overcome these conventional defects, and as a result, they found that β-lactam compounds -- which are synthesized from cycloaddition reactions between diketene and the imine compounds derived from an ester of L-lactic acid -- provide useful intermediates for the synthesis of 4-acetoxy-β-lactam compounds (II). And hence they have completed this invention.

Disclosure of Invention:

This invention concerns the new β-lactam compounds expressed by general formula (I) below and the method of their manufacture:

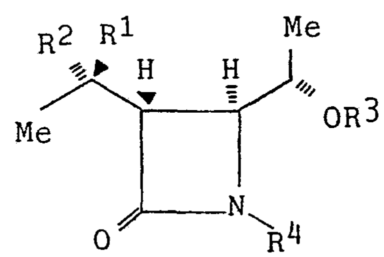

(where $R^1$ and $R^2$ together denote oxo radical, or when $R^1$ or $R^2$

is hydrogen atom the other of the two denotes hydroxyl radical or protected hydroxyl radicals; $R^3$ denotes hydrogen atom or hydroxyl radical-protecting groups; $R^4$ denotes hydrogen atom or amino radical-protecting groups).

Best Mode for Carrying Out the Invention:

The β-lactam compounds of this invention, expressed by general formula (I) above, can be manufactured from the following reaction process, where the key process is cycloaddition reactions between diketene and the imine compounds (VII) that are derived from excessively low-cost esters of L-lactic acid.

(Process 1)

(Process 2)

(Process 3)

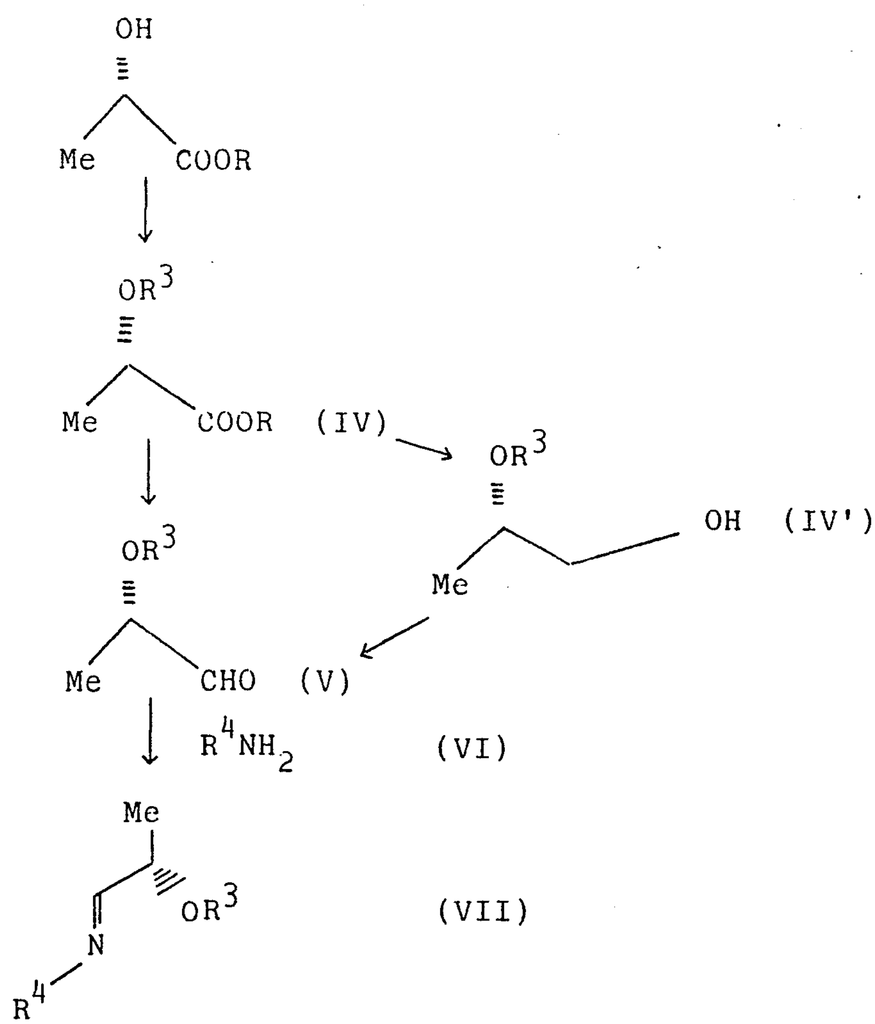

(IV) (IV') (V) (VI) (VII)

(Process 4)

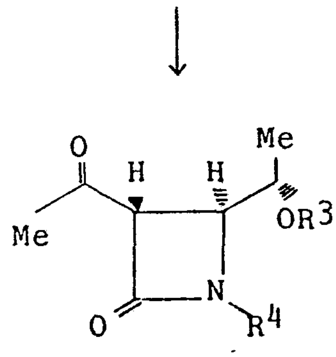

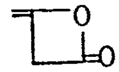

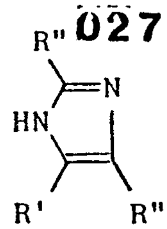

(1a)

(Process 5)

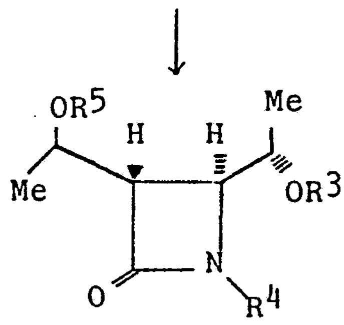

(1b)

(where R denotes hydrogen atom or carboxyl radical-protecting groups; $R^5$ denotes hydrogen atom or hydroxyl radical-protecting groups; R', R" and R'" each denotes hydrogen atom, alkyl radicals or alkenyl radicals, and R' and R" can form a ring by uniting with members to combine with).

(Process 1)

In this process, the hydroxyl radical of an optically active esters of L-lactic acid is protected and L-lactic acid ester derivatives (IV) are therefore manufactured. As the esters of L-lactic acid which are used in this reaction are illustrated methyl L-lactate, ethyl L-lactate, propyl L-lactate, benzyl L-lactate, phenyl L-lactate, etc. which in the succeeding Process-2 can be converted to corresponding

aldehydes or primary alcohols. Further as the hydroxyl radical-protecting groups used can be given trimethylsilyl, triethylsilyl, t-butyldimethylsilyl, t-butyldiphenylsilyl or other trialkylsilyl radicals; methoxymethyl, benzyloxymethyl, t-butoxymethyl, (2-methoxyethoxy)methyl, 1-ethoxyethyl, 2-methoxy-2-propyl, tetrahydropyranyl or other alkoxyalkyl radicals; benzyl, p-methoxybenzyl, 2,4-dimethoxybenzyl, o-nitrobenzyl, p-nitrobenzyl or other substituted or nonsubstituted monoarylmethyl radicals; acetyl, benzoyl, p-methoxybenzoyl, p-nitrobenzoyl or other acyl radicals; benzyloxycarbonyl, p-methoxybenzyloxycarbonyl, p-nitrobenzyloxycarbonyl or other alkoxycarbonyl radicals; allyl radicals or other alkenyl radicals, etc. Protection of these hydroxyl radicals can be accomplished in the normal method ("Protective Groups in Organic Synthesis," John-Wiley & sons, New York, 1981, pp. 10-86) by which hydroxyl radicals are protected.

(Process 2)

In this process, the ester radicals expressed by general formula (IV) and which were obtained in Process 1 are chemically reduced to manufacture the aldehyde derivative expressed by general formula (V). For this process, it is possible to adopt a method where ester radicals are converted to aldehyde radicals in one step, or another method where the ester radicals, expressed by general formula (IV), are first reduced to hydroxymethyl radicals before they are oxidized into aldehyde to synthesize the derivatives expressed by general

formula (V). For the reducing agent used in the conversion in one step, it is possible to use any reducers that are employed in reducing ester radical to corresponding aldehyde radical. Desirably, diisobutylaluminum hydride, sodium bis (2-methoxyethoxy) aluminum hydride, etc. are used. This reaction is allowed to proceed in a solvent, for which normally any solvents may be used unless they participate in the reduction reaction. It is desirable, however, to use diethylether, terahydrofuran, toluene, etc. The reaction proceeds smoothly at a temperature between -100°C and 20°C.

Process 2 can also be effected by another method where ester radicals (IV) are reduced by lithium aluminum hydride, sodium bis (2-methoxyethoxy)aluminum hydride, diisobutylaluminum hydride or other reducing agents to obtain the compounds, expressed by general formula (IV'). On being obtained, these compounds (IV') are oxidized by pyridinium chlorochromate, pyridinium dichromate, dimethylsulfoxide derivatives, etc. to obtain aldehyde derivatives expressed by general formula (V). For solvents used in the reduction or oxidation processes above, normally any solvents may be used unless they participate in the reactions. Preferably, however, diethylether, terahydrofuran, toluene, etc. are used. Under this distinct method, two steps of reaction operations are necessary, but these reactions have the advantage of being attainable at room temperature.

(Process 3)

Under this process, aldehyde derivatives (V) are allowed to react with primary amine compounds expressed by general formula (VI), the water that results as a by-product is then eliminated and thus imine derivatives expressed by general formula (VII) are manufactured. As primary amine compounds (VI) are used those which for substituents expressed by $R^4$, have alkyl radicals, aryl radicals or silyl radicals which can become amino radical-protecting groups. As alkyl radicals can be given benzyloxycarbonylmethyl, p-nitrobenzyloxycarbonylmethyl, methoxycarbonylmethyl, ethoxycarbonylmethyl, t-butoxycarbonylmethyl or other alkoxycarbonylmethyl radicals; benzyl, p-methoxybenzyl, 2,4-dimethoxybenzyl, o-nitrobenzyl, p-nitrobenzyl or other substituted or nonsubstituted monoarylmethyl radicals; diphenylmethyl, di-p-anisylmethyl (di-p-methoxyphenylmethyl), di-p-nitrophenylmethyl or other substituted or nonsubstituted diarylmethyl radicals; trityl or other triarylmethyl radicals, etc. As aryl radicals can be illustrated phenyl, p-anisyl (p-methoxyphenyl), o-anisyl (o-methoxyphenyl), 2,4-dimethoxyphenyl, p-nitrophenyl or other substituted or nonsubstituted aryl radicals. As silyl radicals can be illustrated trimethylsilyl, triethylsilyl, t-butyldimethylsilyl, t-butyldiphenylsilyl or other trialkylsilyl radicals.

For the method of dehydration under this process, a method based upon azeotropic distillation with a solvent or another dehydrator-based method can be used, either singly or in combination. For the dehydrating agent may be used any

dehydrators unless they trigger reactions other than dehydration in a reaction system. Desirably, however, anhydrous sodium sulfate, anhydrous magnesium sulfate, calcium chloride, Molecular Sieves, etc. are used. for solvents can be illustrated methanol, ethanol, propanol, butanol or other alcohol solvents; benzene, toluene, cyclohexane or other hydrocarbon solvents; diethyl ether, tetrahydrofuran or other ether solvents; chloroform, dichloromethane, carbon tetrachloride or other halogenated hydrocarbon solvents. The reaction proceeds smoothly at a temperature between -20°C and 100°C.

(Process 4)

Under this process, diketene and the imine derivatives (VII) obtained in Process 3 are allowed to react with each other in the presence of imidazole derivatives and thus β-lactam compounds expressed by general formula (1a) are manufactured. For imidazole derivatives may be given imidazole, 2-methylimidazole, 4-methylimidazole, 2,4-dimethylimidazole, 2,4,5-trimethylimidazole, 4-arylimidazole, benzimidazole, etc., but the use of imidazole is preferred. Imidazole derivatives may be used in an amount equivalent to 0.1 to 5 times the amount of imine derivatives, but for improved yields one equivalent is desired. Preferably, this process is performed in a solvent, for which can be given benzene, toluene, xylene or other aromatic hydrocarbons; diethylether, tetrahydrofuran or other ethers; chloroform,

dichloromethane, carbon tetrachloride or other halogenated hydrocarbon; acetonitrile, dimethylformamide, dimethylsulfoxide, hexamethylphophorictriamide or other polar solvents. The reaction goes smoothly at a temperature between -50°C and 100°C.

(Process 5)

In this process, the ketone portion of β-ketolactam derivatives (1a) is chemically reduced to manufacture corresponding alcohol products expressed by general formula (1b). For the reducing agent, any reducing agents may be used that are normally used when ketone is reduced to synthesize corresponding secondary alcohol. Desirably, however, potassium tri-sec-butylborohydride, potassium triethylborohydride, borane pyridine complexes, borane diisopropylamine complexes, etc. are used. for the solvents can be used water, methanol, propanol, butanol or other proton solvents; benzene, toluene or other aromatic hydrocarbon solvents; diethylether, tetrahydrofuran, dioxane or other ether solvents; chloroform, dichloromethane, carbon tetrachloride or other halogenated hydrocarbon solvents; dimethylsulfoxide, hexamethylphophorictriamide or other polar solvents. The reaction proceeds smoothly at a temperature between -80°C and 100°C.

Concerning the hydroxyl radicals at the 3-position of the side chain of the β-lactam (1b) obtained in the above reaction process, it is possible to introduce thereto a protecting group in a method by which hydroxyl radicals are normally protected (Protective Groups in Organic Synthesis, John-Wiley & Sons, new

York, 1981, pp. 10-86). As the hydroxyl radical-protecting groups used can be illustrated trimethylsilyl, triethylsilyl, t-butyldimethylsilyl, t-butyldiphenylsilyl or other trialkylsilyl radicals; methoxymethyl, methylthiomethyl, benzyloxymethyl, t-butoxymethyl, (2-methoxyethoxy)methyl, 1-ethoxyethyl, 2-methoxy-2-propyl, tetrahydropyranyl or other alkoxyalkyl radicals; benzyl, p-methoxybenzyl, 2,4-dimethoxybenzyl, o-nitrobenzyl, p-nitrobenzyl or other substituted or nonsubstituted monoarylmethyl radicals; acetyl, benzoyl, p-methoxybenzoyl, p-nitrobenzoyl or other acyl radicals; benzyloxycarbonyl, p-methoxybenzyloxycarbonyl, p-nitrobenzyloxycarbonyl or other alkoxycarbonyl radicals. Further regarding $R^3$, which is a hydroxyl radical-protecting group introduced to the 4-position on the side chain of β-lactam (1b) in Process 1, and concerning $R^4$, which is an amine radical-protecting group introduced to the 1-position as a substituent of primary amine compounds (VI), they can be readily converted to free hydroxyl radicals or amino radicals in a normal method by which a protection is canceled (Protective Groups in Organic Synthesis, John-Wiley & Sons, New York, 1981).

In the following, this invention is explained in detail using working examples and reference examples, which however in no way limit the scope of this invention. The abreviations used mean as follows.

Ac : Acetyl radical $(-\underset{\underset{O}{\|}}{C}-CH_3)$

0271586

Bn : Benzyl radical ($-CH_2-$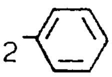 )

DAM : Di-p-anisylmethyl radical (-CH 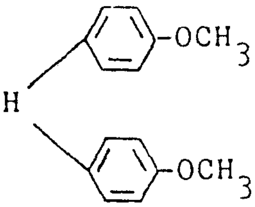 )

Et : Ethyl radical ($-C_2H_5$)

ImH : Imidazole ( 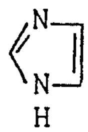 )

Me : Methyl radical ($-CH_3$)

TBDMS : t-butyldimethylsilyl radical ( 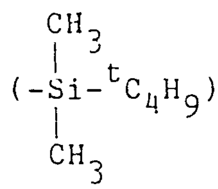 )

THF : Tetrahydrofuran ( 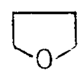 )

Reference example 1

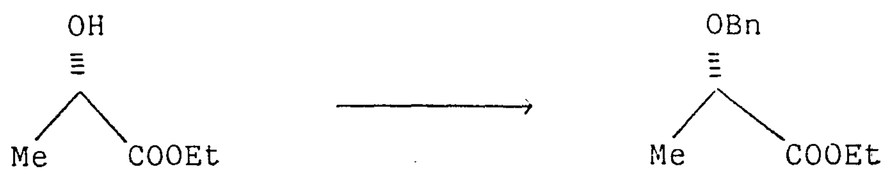

3.50 g of (L)-ethyl lactate was dissolved in a mixed solvent of 70 ml of cyclohexane and 10 ml of dichloromethane, to which was then added 11 ml of benzyl 2,2,2-trichloroacetimidate. Further 1 ml of trifluoromethanesulfonic acid was added at room temperature and the mixture stirred for 20 hours at the same temperature. The mixed liquid was further fed with 20 ml of water and 80 ml of n-hexane and stirred for 3 hours, at the end of which an insoluble material was filtered out for removal. The organic layer was separated and washed

with saturated sodium carbonate solution and a saturated sodium chloride solution. After it was dried over magnesium sulfate, the solvent was distilled off under reduced pressure, the residue obtained was refined with a column of silica gel. As a result, 4.27 g (yield 69%) of ethyl (S)-2-benzyloxypropionate was obtained.

Form : colorless oil

$(\alpha)_D^{20}$ -74.5° (c=2.94, $CHCl_3$)

IR (neat) : 1750, 1455, 1271, 1200, 1142, 1067, 1027, 740, 702 $mc^{-1}$.

Mass m/e : 179 $(M-29)^+$, 102 $(m-106)^+$.

H-NMR ($CDCl_3$) δ=1.29 (3H, t, J=7.1Hz), 1.43 (3H, d, J=6.8Hz), 4.05 (1H, q, J=6.8Hz), 4.22 (2H, q, J=7.1Hz), 4.57 (2H, dd, J=11.6, 24.4Hz), 7.33 (5H, s).

Reference example 2

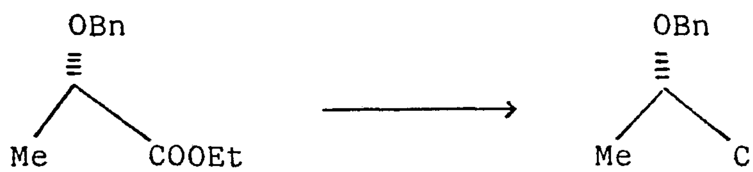

373 mg of ethyl (S)-2-benzyloxypropionate was dissolved in 9 ml of anhydrous ether, to which was then slowly added dropwise, at -78°C, a 2.7 ml of 1M hexane solution of diisobutylaluminumdihydride. Ten minutes after this, the mixed liquid was fed with 0.1 ml of methanol, the reaction was stopped, again 0.27 g of water was added, and the liquid was stirred for 1 hour at room temperature. An insoluble material was filtered out through a column of celite, the filtrate was

then condensed and, at the end of this, was refined with a column of silica gel (hexane: ethyl acetate = 47:3-9:1) into 241 mg of (S)-2-benzyloxypropanal. (Yield 82%)

Form : Colorless oil

$(\alpha)_D^{20}$-40.9° (c=9.99, $CHCl_3$)

1R (neat) : 1740, 1500, 1456, 1380, 1210, 1100, 741, 702 cm$^{1}$.

Mass m/e : 135 $(M-29)^+$.

H-NMR ($CDCl_3$) δ=1.32 (3H, d, J=6.8Hz), 3.88 (1H, dq, J=1.8, 6.8Hz), 4.62 (2H, s), 7.35 (5H, s), 9.66 (1H, d, J=1.8Hz).

Reference example 3

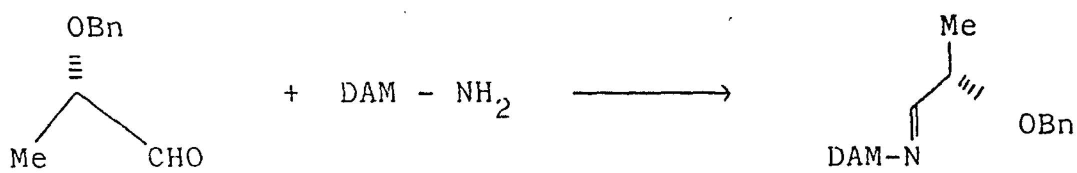

0.737 g (4.55 mmol) of (S)-2-benzyloxypropanal was dissolved in 10 ml of toluene, to which was then added 1.11 g (4.55 mmol) of di-p-anisylmethylamine and 1.09 g of anhydrous magnesium sulfate, the mixture then being stirred for 1 hour at 0°C. An insoluble material was eliminated by filtration, after which the solvent was distilled off at reduced pressure and thus (S)-N-(2-benzyloxy-1-propylidene) di-p-anisylmethylamine was obtained in a quantitative yield. since being unstable, this product was immediately used in reactions in example 1-1.

H-NMR ($CDCl_3$) δ=1.36 (3H, d, J=6.4Hz), 3.78 (3H, s), 3.79

(3H, s), 4.17 (1H, m), 4.51 (2H, s), 5.34 (1H, s), 6.84 (4H, d, J=9.0Hz), 7.29 (5H, s).

Example 1-1

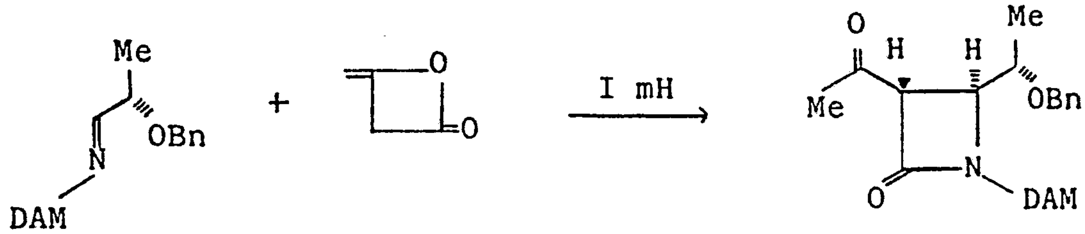


The (S)-N-(2-benzyloxy-1-propylidene) di-p-anisylmethylamine obtained in reference example 3 was dissolved in 18.2 ml of THF, to which was then introduced 369 mg of imidazole, the mixture being then cooled to -35°C and fed dropwise, over a period of 3 hours, with a solution of 1.78 ml of diketene in 18.2 ml of THF solution. the mixed solution was further stirred at the same temperature for 4 consecutive days, at the end of which the liquid was fed with 10 ml of a 0.1N hydrochloric acid solution and stirred for 2 hours at room temperature. Again fed with 120 ml of ethyl acetate, the liquid was separated into two layers, the organic layer being then twice washed with 10 ml of a saturated sodium chloride solution and dried over magnesium sulfate. The residue was separated and purified with a column of silica gel (dichloromethane : acetone=49:1), and thus (3S, 4S, 1'S)-1-(di-p-anisylmethyl)-3-acetyl-4-(1'-benzyloxyethyl)-azetidine-2-one was obtained in an amount of 1.55 g (72%).

Form : Colorless caramel.

0271586

$(\alpha)_D^{20}$-7.3° (c=1.48, $CHCl_3$)

1R (neat) : 2950, 1760, 1720, 1614, 1589, 1514, 1460, 1306, 1250, 1190, 1099, 1036, 828, 740, 702, 578 $cm^{-1}$.

Mass m/e : 473 ($M^+$), 382 $(M-91)^+$.

H-NMR ($CDCl_3$) δ=1.09 (3H, d, J=6.4Hz), 2.26 (3H, s), 3.74 (1H, bs), 3.76 (3H, s), 3.78 (3H, s), 4.29 (2H, dd, J=11.4, 23.8Hz), 5.75 (1H, s), 6.79 (4H, d, J=7.2Hz), 7.03-7.31 (9H, m).

The optical purity of the product was determined by measuring its H-NMR using tris(3-(heptafluoropropylhydroxymethylene)-d-camphorato) europium (III) derivative, $(Eu(hfc)_3)$, an optically active shift reagent. As a result, the optical purity of the product was found to be 96% ee.

Example 1-2

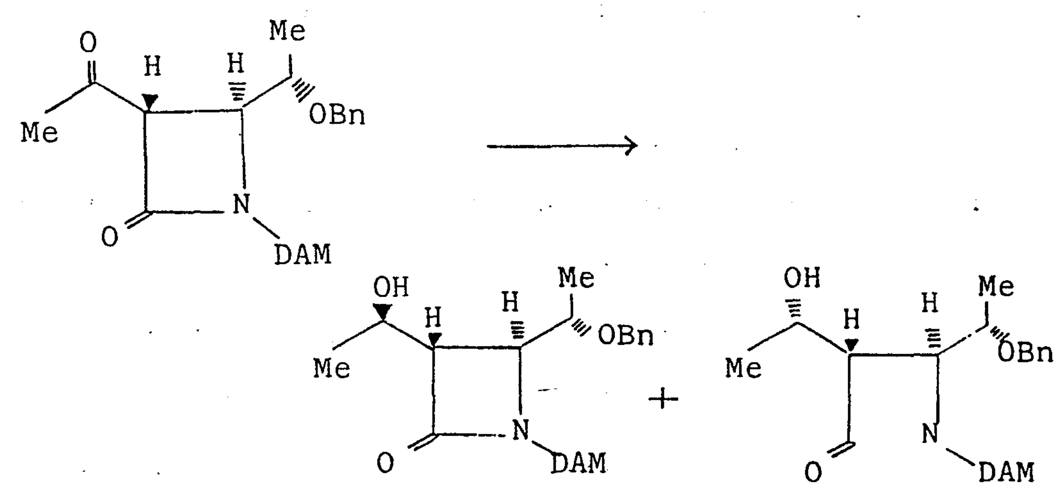

A solution of 138 mg (0.29 mmol) of (3S, 4S, 1'S)-1-(di-p-anisylmethyl)-3-acetyl-4-(1'-benzyloxyethyl)azetidine-2-one and 48 mg (0.29 mmol) of potassium iodide in 2.9 ml of THF solution

was supplied with 0.5 ml of a 1M THF solution of K-Selectride (a registered trade mark) and was stirred for 30 minutes. To this were then added 0.5 ml of 1N hydrochloric acid and 20 ml of ethyl acetate; the mixed liquid was then separated into two layers, of which the organic layer was then washed with a saturated sodium chloride solution and was dried over magnesium sulfate, the solvent being thereafter distilled off. The residue obtained was refined with a column of silica gel (dichloromethane : acetone=1:0 ⟶ 9:1) and consequently a mixture of (3S, 4S, 1'R, 1"S)- and (3S, 4S, 1'S, 1"S)-1-(di-p-anisylmethyl)-3-(1'-hydroxyethyl)-4-(1'-benzyloxyethyl)-azetidine-2-one was produced in an amount of 118 mg (85% yield). This product was measured for H-NMR spectrum in benzene-d6 and as a result the signals of the 1'-position and of 1"-position methyl of the (3S, 4S, 1'R, 1"S)-form stood at 0.94 ppm (d, J=6.2Hz) and 1.19 ppm (d, J=6.2Hz), respectively, and those of (3S, 4S, 1'S, 1"S)-product, at 0.86 ppm (d, J=5.9Hz) and 1.15 ppm (d, J=6.4Hz). From the intensity ratio of these signals, their formation ratio was determined at approximately 10:1. when this mixture was measured for H-NMR spectrum in determined chloroform, it was found to be almost the same with that of pure (3S, 4S, 1'R, 1"S) form given below. This diastereomer mixture was further separated using silica gel TLC (ether) and as a result, (3S, 4S, 1'R, 1"S)-1-(di-p-anisylmethyl)-3-(1'-hydroxyethyl)-4-(1"-benzyloxyethyl)-azetidine-2-one was obtained in an amount of 106 mg.

Form : Colorless caramel.

H-NMR ($CDCl_3$) δ=1.13 (3H, d, J=5.9Hz), 1.25 (3H, d,

J=6.4Hz), 2.86 (1H, dd, J=2.2, 5.9Hz), 3.49 (1H, m), 3.76 (3H, s), 3.78 (3H, s), 4.30 (2H, dd, J=13.4, 31.4Hz), 5.77 (1H, s), 6.79 (4H, d, J=8.5Hz), 7.0-7.3 (9H, m).

Example 1-3

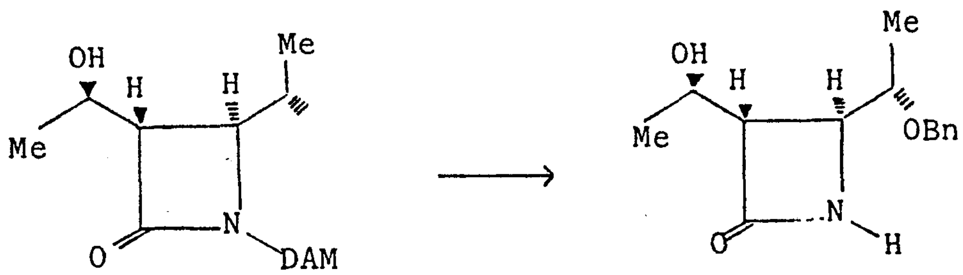

38.7 mg (0.081 mmol) of (3S, 4S, 1'R, 1'R, 1"S)-1-(di-p-anisylmethyl)-3-acetyl-4-(1'-hydroxyethyl-4-(1"-benzyloxyethyl)-azetidine-2-one was dissolved in 0.6 ml of a 10% water-acetonitrile solution, to which was then added 0.8 ml of cerium (IV) ammonium nitrate. The mixture was then stirred for 30 minutes at room temperature and, after this, was fed with 1 ml of a saturated sodium bicarbonate solution. Organic substances were extracted with chloroform and dried over magnesium sulfate, then the solvent was distilled off under reduced pressure, and the residue obtained was separated and purified with a column of silica gel (dichloromethane: acetone=4:1-2:1). Consequently, 15.3 mg (yield 75%) of (3S, 4S, 1'R, 1"S)-3-(1'-hydroxyethyl)-4-(1"-benzyloxy-ethylazetidine-2-on was obtained.

Form : Colorless crystals.

Melting point : 129-130°C.

H-NMR ($CDCl_3$) δ=1.25 (3H, d, J=5.9Hz), 1.31 (3H, d, J=6.4Hz), 2.86 (1H, m), 3.58 (2H, m), 4.16 (1H, m), 4.55 (2H, dd, J=11.4, 25.1Hz), 5.95 (1H, bs), 7.25 (1H, s).

Example 1-4

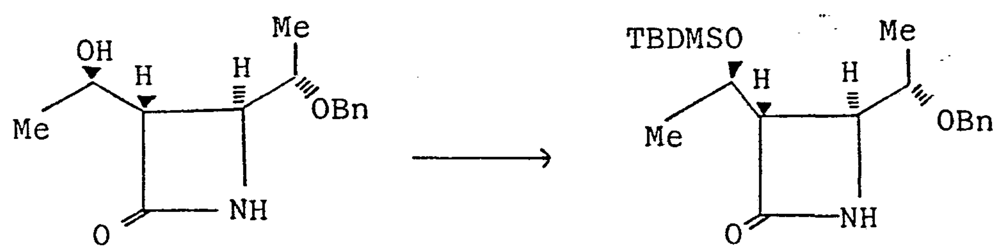

6.5 mg (0.026 mmol) of 3S, 4S, 1'R, 1"S)-3-(1'-hydroxyethyl)-4-(1"-benzyloxyethyl)-azetidine-2-one and 3.5 mg (0.052 mmol) of imidazole were dissolved in 0.2 ml of DMF and 7.8 mg (0.052 mmol) of t-butyldimethylchlorosilane was then added to this solution, which thereafter being stirred overnight at room temperature. With water and ethyl acetate added to it, the solution was then separated into two layers, the organic layer being washed with water and dried over magnesium sulfate.

The solvent was distilled off at reduced pressure and the residue obtained was purified with a column of silica gel (dichloromethane: acetone=1:0-19:1). (3S, 4S, 1'R, 1"s)-3-(1'-t-butyldimethylsilyloxyethyl)-4-(1"-benzyloxyethyl)-azetidine-2-one was thus obtained in an amount of 9.2 mg (yield 97%).

Form : Colorless caramel

H-NMR ($CDCl_3$) δ=0.07 (6H, s), 0.87 (9H, s), 1.22 (3H, d, J=6.2Hz), 1.25 (3H, d, J=5.9Hz), 2.74 (1H, m), 3.52 (2H, m), 4.17 (1H, quint, J=6Hz), 4.47 (2H, dd, J=11.7, 24.8Hz), 5.87 (1H, bs), 7.25 (5H, s).

Example 1-5

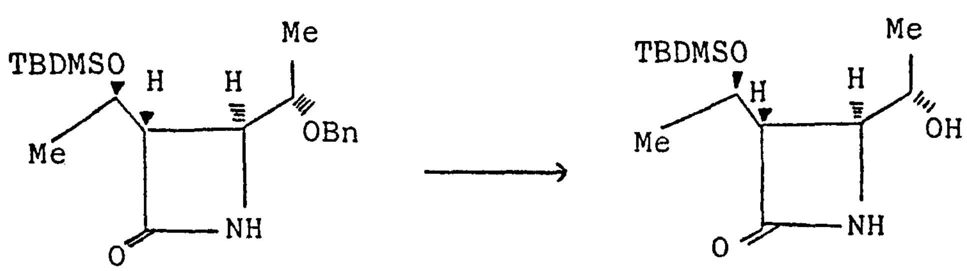

7.1 mg of (3S, 4S, 1'R, 1"S)-3-(1'-t-butyldimethylsilyloxyethyl)-4-(1"-benzyloxyethyl)-azetidine-2-one was dissolved in 1 ml of ethyl acetate, to which was then added 1 mg of 5% palladium-carbon, the mixture being stirred overnight at room temperature in a hydrogen atomosphere. the catalyst was removed with a column of celite, the solvent distilled off, and thus 5.3 mg (99% yield) of (3S, 4S, 1'R, 1"S)-3-(1'-t-butyldimethylsilyoxyethyl)-4-(1"-hydroxyethyl)-azetidine-2-one was obtained.

Form : Colorless caramel

H-NMR ($CDCl_3$) δ=0.08 (6H, s), 0.88 (9H, s), 1.23 (3H, d, J=6.2Hz), 1.25 (3H, d, J=6.2Hz), 2.85 (1H, m), 3.55 (1H, dd, J=2.2, 6.8Hz), 3.72 (1H, m), 4.20 (1H, quint, J=6.2Hz), 6.11 (1H, s).

Reference example 4

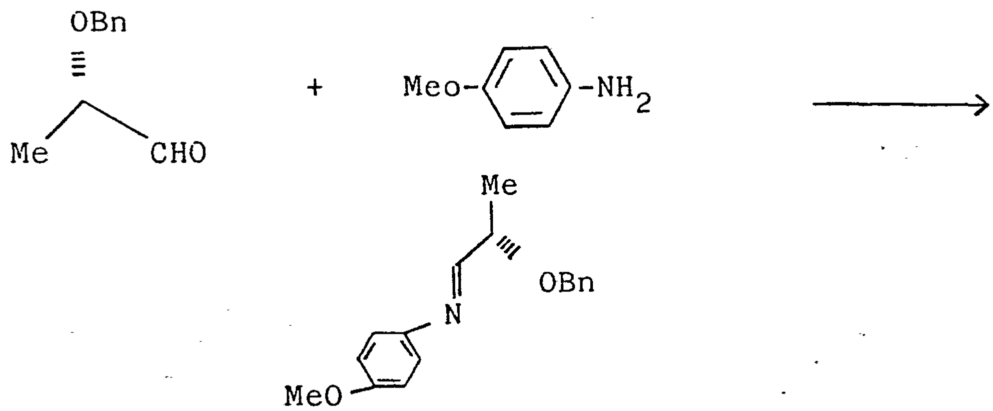

236 mg (1.46 mmol) of (S)-2-benzyloxypropanal was dissolved in 2 ml of toluene, to which was then added 178 mg (1.46 mmol) of p-anisidine and 174 mg anhydrous magnesium sulfate, the mixture being then stirred for 1 hour at 0°C. After insoluble materials were filtered off, the solvent was distilled off under reduced pressure and, as a result, (S)-N-(2-benzyloxy-1-propylidene)-p-anisylamine was obtained in a quantitative yield. Since it is unstable, the product was immediately used in reactions in example 2.

N-NMR ($CDCl_3$); δ=1.43 3H, d, J=6.6Hz), 3.81 (3H, s), 4.19 (1H, dq, J=5.5, 6.6Hz), 4.61 (2H, s), 6.86 (2H, d, J=9.2Hz), 7.06 (2H, d, J=9.2Hz), 7.33 (5H, s), 7.79 (1H, d, J=5.5Hz).

Example 2

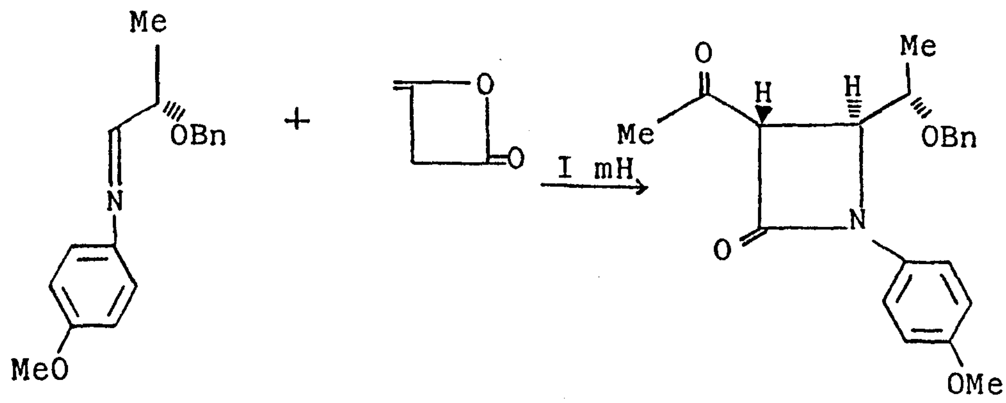


The (S)-N-(2-benzyloxy-1-propylidene)-p-anisylamine was dissolved in 2.9 ml of THF. After 99 mg of imidazole was added to it, the solution was cooled to -30°C and was fed drop by drop with a solution of 0.34 ml of diketene in 2.6 ml of THF. The solution was stirred for 3 days at the same temperature, after which it was fed with 1 ml of a solution of 1N hydrochloric acid and stirred for 2 hours at room temperature. With ethyl acetate added to it, the mixture was separated into two layers, the organic layer being washed with a saturated sodium chloride solution, an aqueous saturated solution of sodium bicarbonate and a saturated sodium chloride solution in that order, and dried over magnesium sulfate. The solvent was distilled off under reduced pressure, the residue was separated and purified with a column of silica gel (dichloromethane: acetone=1:0-49:1) and as a result 133 mg (yield 26%) of (3S, 4S, 1'S)-1-(p-anisyl)-3-acetyl-4-(1'-benzyloxyethyl)-azetidine-2-one was obtained.

Form : Oil

$(\alpha)_D^{20}$ $-14.0°$ (c=0.71, $CDCl_3$).

IR (neat) : 2950, 1753, 1720, 1514, 1360, 1249, 1115, 1029, 830, 740, 700 $cm^{-1}$.

Mass m/e 353 ($M^+$).

H-NMR ($CDCl_3$) δ=1.19 (3H, d, J=6.2Hz), 2.35 (3H, s), 3.77 (3H, s), 3.91 (1H, quint, J=6.3Hz), 4.03 (1H, d, J=2.5Hz), 4.50 (2H, dd, J=11.8, 17.5Hz), 4.63 (1H, dd, J=2.5, 6.3Hz), 6.81 (2H, d, J=9.2Hz), 7.25 (5H, s), 7.34 (2H, d, J=9.2Hz).

Example 3

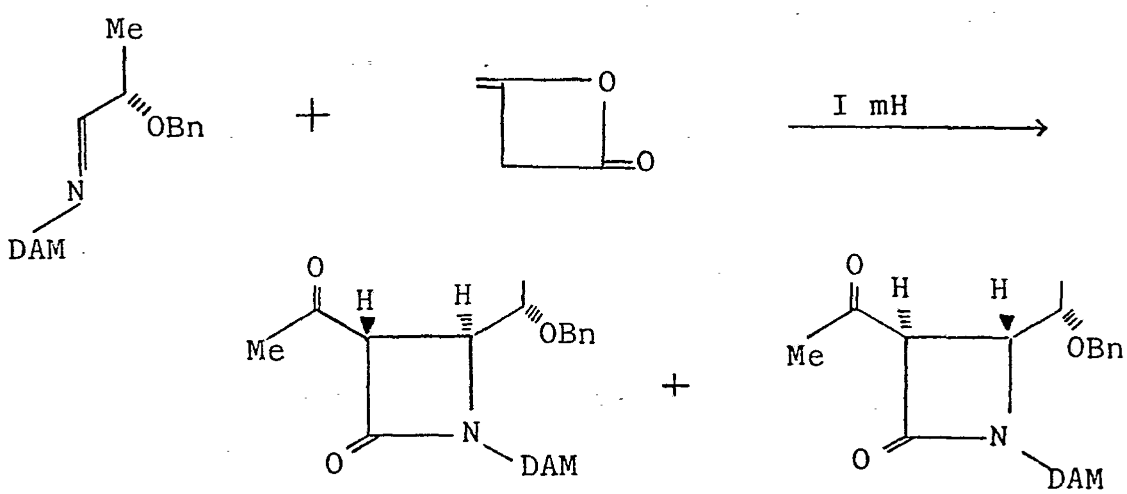


0.500 g (1.29 mmol) of (S)-N-(2-benzyloxy-1-propylidene) di-p-anisylmethylamine was dissolved in 18 ml of THF. After 87.5 mg (1.29 mmol) of imidazole was added to it, the solution was cooled to -30°C and fed dropwise for 3 hours with 0.302 ml (3.85 mmol) of diketene. Again the mixture was stirred for 1 day at the same temperature before it was fed with 2 ml of an aqueous solution of 1N hydrochloric acid. An organic substance

was extracted with ether, the organic layer was washed sequentially with a saturated sodium chloride solution, an aqueous solution of 1N sodium hydroxide and a saturated sodium chloride solution in that order. The solvent was distilled off under reduced pressure and as a result a crude mixture of (3S, 4S, 1'S)- and (3R, 4R, 1'S)-1-(di-p-anisylmethyl)-3-acetyl-4-(1'-benzyloxyethyl)-azetidine-2-one was obtained. This mixture was measured for H-NMR spectrum in chloroform-d and it was found that the signal of acetyl at the 3-position of (3S, 4S, 1'S)-product stood at 2.26 ppm (singlet) and that of (3R, 4R, 1'S)-product at 2.32 ppm (singlet). From their signal intensity ratio, it was determined that the formation ration was 20:1.

This diastereomeric mixture was separated and purified with a column of silica gel (hexane : ethyl acetate = 17:3 ⟶ 3:2) and thus 0.336 g (57%) of (3S, 4S, 1'S)-product was obtained.

This product agreed with the one obtained in example 1-1 in the optical rotation value and in a variety of IR, NMR and Mass spectra.

Examples 4 and 5

By following the same procedures as example 3, mixtures (3S, 4S, 1'S)-product (A) and (3R, 4R, 1'S)-product (B) of β-lactam compounds were obtained from (S)-N-(2-benzyloxy-1-propylidene) di-p-anisylmethylamine. The reaction conditions and the results are shown in the table below.

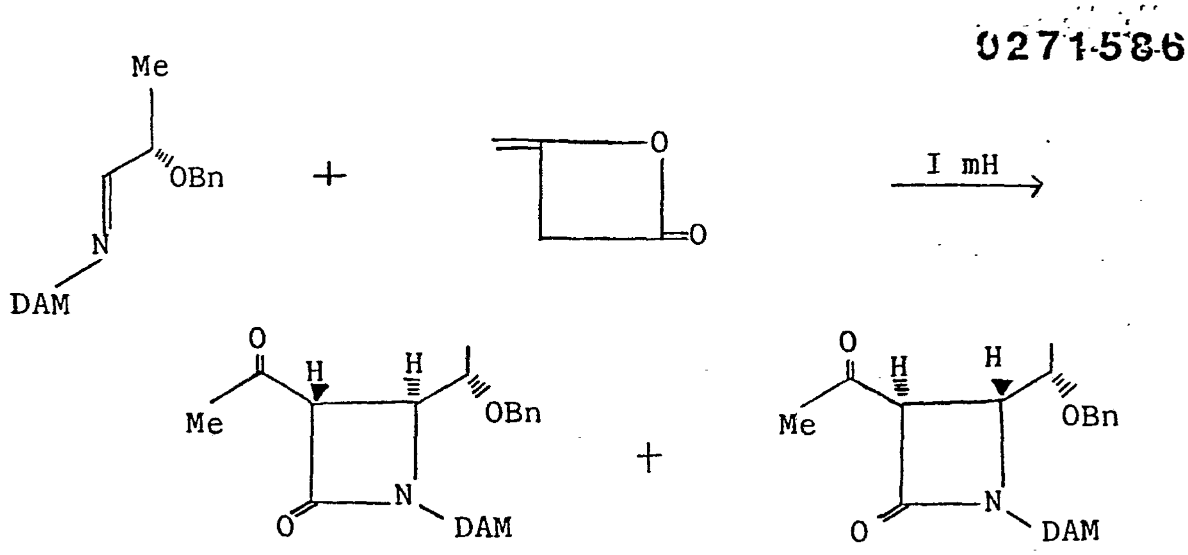


A B

| Working example No. | Solvent | Ratio A:B | Yield of A |
|---|---|---|---|
| 4 | $CH_3CN$ | 10:1 | 61% |
| 5 | $CH_2Cl_2$ | 11:1 | 59% |

Industrial Applicability :

By the synthesizing process given below, β-lactam compounds expressed by general formula (1) can be chemically led to 4-acetoxy-β-lactam derivatives expressed by general formula (II).

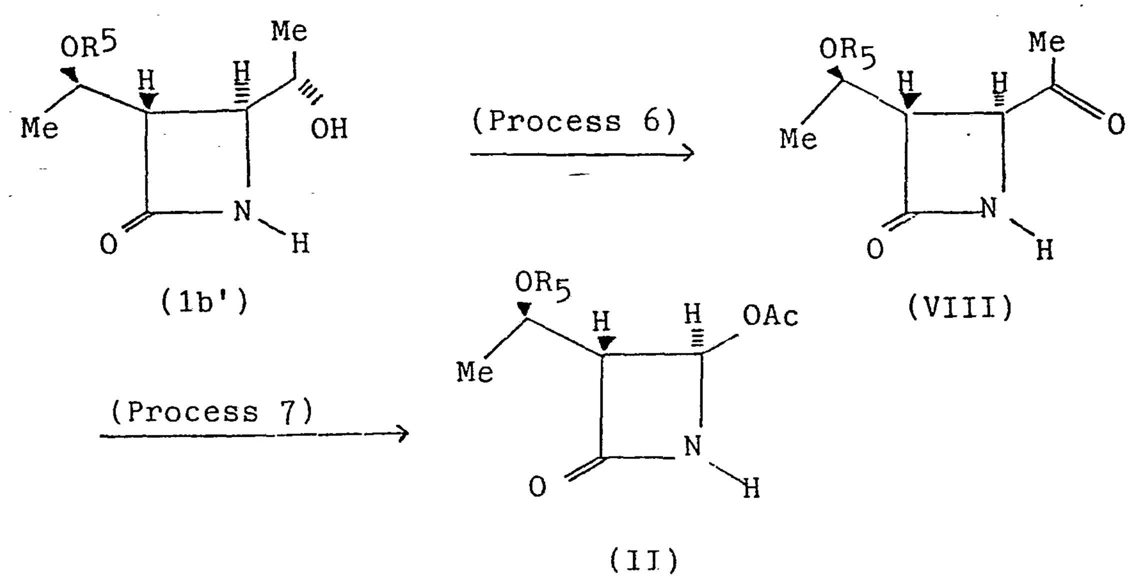


(1b') (VIII) (II)

(Process 6)

In this process, the secondary alcohol on the side chain at the 4-position of the β-lactam derivatives (1b') obtained in process 5, is oxidized to synthesize the 4-acetyl-β-lactam expressed by general formula (VIII). for the oxidizing agent, any oxidants that can oxidize ordinary secondary alcohol into ketone may be used, but preferably chromium trioxide, pyridinium chlorochromate, pyridinium dichromate or other chromic acids; dimethylsulfoxide derivatives and ruthenium compounds are used. Desirably this reaction should go in a solvent, for which can be illustrated pyridine, collidine, lutidine or other basic solvents; dimethylsulfoxide, N,N-dimethylformamide, N,N-dimethylacetoamide, hexamethylphosphorictriamide or other polar aprotonic solvent; dichloromethane, chloroform, carbon tetrachloride or other halogenated hydrocarbon solvent; benzene, toluene, hexane or other hydrocarbon solvents; diethyl ether, dibutyl ether, tetrahydrofuran, dioxane or other ether solvents. The reaction proceeds smoothly at a temperature between -78°C and 150°C.

(Process 7)

In this process, a known method (M. Shiozaki et al, Tetrahedron, 39, 2399 (1983) and T. Chiba et al, Chem. Lett., 1985, 651.) is followed to treat with a peracid the 4-acetyl-δ-lactam (VIII), which was obtained in Process 6, and to thereby lead it to 4-acetoxy-β-lactam. As the peracid can be illustrated m-chloroperbenzoic acid, peracetic acid, perphthalic acid, etc. As the solvent used can be illustrated

benzene, toluene, hexane or other hydrocarbon solvents; diethyl ether, tetrahydrofuran, dioxane, 1,2-dimethoxyethane or other ether solvents; chloroform, dichloromethane, carbon tetrachloride or other halogenated hydrocarbon solvents; ethyl acetate, methyl acetate or other ester solvents. The reaction proceeds smoothly at a temperature between 0°C and 50°C.

Reference example 5

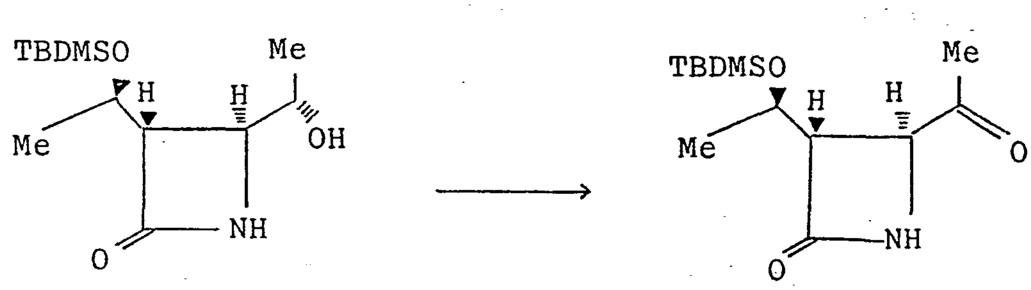

2.1 mg of (3S, 4S, 1'R, 1"S)-3-(1'-t-butyldimethylsilyl-oxyethyl)-4-(1"-hydroxyethyl)-azetidine-2-one was dissolved in 0.2 ml of pyridine and 0.3 ml of dichloromethane. This solution was then supplied with 77 mg of chromium trioxide and stirred for 1 hour at room temperature. The reaction liquid was then passed through a column of silica gel (dichloromethane: acetone=1:0 ⟶ 4:1) to remove inorganic salts and after this, under reduced pressure, the solvent was distilled off to obtain (3S, 4S, 1'R)-3-(1'-t-butyldimethylsilyloxyethyl)-4-acetyl-azetidine-2-on in an amount of 2.1 mg

(quantitative yield).

Form : Colorless caramel

H-NMR ($CDCl_3$) δ=0.11 (6H, s), 0.90 (9H, s), 1.31 (3H, d, J=6.4Hz), 2.25 (3H, s), 3.08 (3H, m), 4.28 (2H, m), 6.05 (1H, bs).

Reference example 6

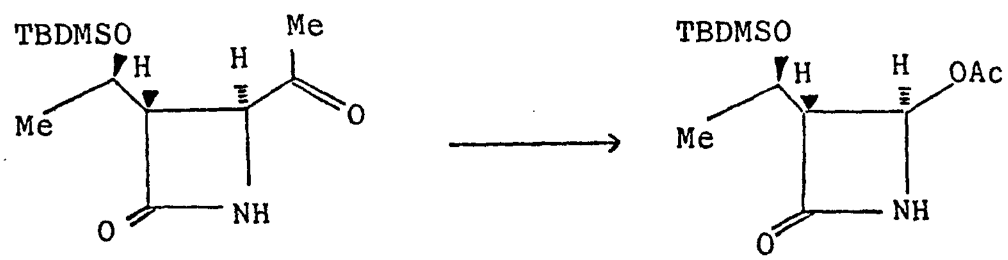

1.9 mg of (3S, 4S, 1'R)-3-(1'-t-butyldimethylsilyloxyethyl)-4-acetyl-azetidine-2-on was dissolved in 0.2 ml of ethyl acetate, to which was then added 10 mg of metachloroperbenzoic acid. The mixture was then stirred for 2 hours at 35°C; diluted with ethyl acetate; washed with 0.4 ml of an aqueous solution of sodium hydrogensulfite, an aqueous solution of sodium bicarbonate, and a saturated sodium chloride solution in that order, and dried over magnesium sulfate, The solvent was distilled off under reduced pressure and the residue obtained was purified with a column of silica gel (hexane: ethyl acetate=7:3) and thus (3R, 4R, 1'R)-3-(1'-t-butyldimethylsilyloxyethyl)-4-acetoxy-azetidine-2-one was obtained in an

amount of 2.0 mg (quantitative yield).

Form : Colorless crystal

Melting point : 107-108°C (values mentioned in literature: 170-108°C; W. J. Leanza et al, Tetrahedron, 39, 2505 (1983).)

IR (KBr) : 2950, 1787, 1746, 1235, 1164, 1080, 1040, 839, 779 $cm^{-1}$

Mass m/s : 230 $(M-57)^+$

$(\alpha)_D^{20}$ + 48.2° (c=0.19, $CHCl_3$)

(values mentioned in literature: $(\alpha_D^{20}$ + 50.0° (c=0.41, $CHCl_3$); W. J. Leanza et al, Tetrahedron, 39, 2505 (1983).)

H-NMR ($CDCl_3$); δ=0.07 (6H, s), 0.87 (9H, s), 1.26 (3H, d, J=6.4Hz), 2.11 (3H, s), 3.18 (1H, dd, J=1.3, 3.5Hz), 4.23 (1H, dq, J=3.5, 6.4Hz), 5.84 (1H, bs, 6.51 (1H, bs).

# CLAIMS

1. The β-lactam compounds expressed by the general formula below:

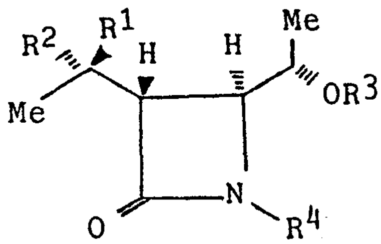

(where $R^1$ and $R^2$ together denote oxo radical, or when $R^1$ or $R^2$ is hydrogen atom the other of the two denotes hydroxyl radical or protected hydroxyl radical; $R^3$ denotes hydrogen atom or hydroxyl radical-protecting groups; $R^4$ denotes hydrogen atom or amino radical-protecting groups).

2. The method of manufacturing the β-lactam compounds which are expressed by a general formula below:

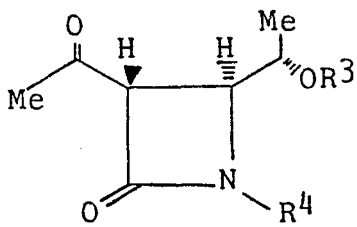

(where $R^3$ denotes hydrogen atom or hydroxyl radical-protecting groups; $R^4$ denotes hydrogen atom or amino radical-protecting groups) and whose feature it is to allow reactions to take place between diketene and the imine derivatives, expressed by a general formula below,

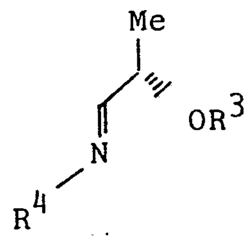

in the presence of the imidazole derivatives expressed by another general formula below:

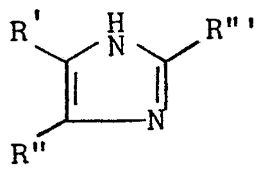

(R', R" and R'" each denote hydrogen atoms, alkyl radicals or alkenyl radicals, and R' and R" can form a ring by uniting with members to combine with)

# INTERNATIONAL SEARCH REPORT

0271586

International Application No PCT/JP87/00354

**I. CLASSIFICATION OF SUBJECT MATTER** (if several classification symbols apply, indicate all) [3]

According to International Patent Classification (IPC) or to both National Classification and IPC

Int.Cl$^4$ C07D205/08

**II. FIELDS SEARCHED**

Minimum Documentation Searched [4]

| Classification System | Classification Symbols |
|---|---|
| IPC | C07D205/08 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| X | JP, A, 60-32765 (Sumitomo Chemical Co., Ltd.) 19 February 1985 (19. 02. 85) Page 1, & US, A, 4556514 & EP, A1, 106652 & ES, A1, 526759 | 1-2 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| August 21, 1987 (21. 08. 87) | September 7, 1987 (07. 09. 87) |
| **International Searching Authority [1]** | **Signature of Authorized Officer [20]** |
| Japanese Patent Office | |